(19) Europäisches Patentamt
European Patent Office
Office européen des brevets

(11) Publication number: **0 283 270 A2**

(12) **EUROPEAN PATENT APPLICATION**

(21) Application number: 88302305.3

(22) Date of filing: 16.03.88

(51) Int. Cl.4: C 07 F 9/54
C 07 B 39/00

(30) Priority: 16.03.87 US 26311 30.04.87 US 44162
04.06.87 US 58107 22.01.88 US 146944

(43) Date of publication of application:
21.09.88 Bulletin 88/38

(84) Designated Contracting States:
AT BE CH DE ES FR GB GR IT LI LU NL SE

(71) Applicant: ICI AMERICAS INC
Concord Pike & New Murphy Road
Wilmington Delaware 19897 (US)

(72) Inventor: Timony, Peter Edward
562 Babbling Brook Lane
Valley Cottage, New York 10989 (US)

Bay, Elliott
20 Woodland Way
Ridgefield, Connecticut 06877 (US)

Leone-Bay, Andrea
20 Woodland Way
Ridgefield, Connecticut 06877 (US)

(74) Representative: Froud, Clive et al
Elkington and Fife High Holborn House 52/54 High Holborn
London WC1V 6SH (GB)

(54) **Processes using a phosphorus complex.**

(57) A process for the preparation of a phosphorus complex corresponding to the following general formula:

$$\overset{\oplus}{RPCl_3}\overset{\ominus}{PCl_6}$$

wherein R represents optionally substituted aryl, wherein the substitution may comprise at least one selected from nitro, chloro, fluoro, alkyl, fluoro-alkyl and alkoxy, or optionally substituted alkyl; characterised in that it comprises reacting phosphorus pentachloride and a phosphonic dichloride corresponding to the following general formula:

$RPOCl_2$

wherein R is as defined above; in the presence of an inert solvent boiling at a temperature of from 120 to 150°C, which is capable of co-distilling a phosphorus oxychloride reaction product, and co-distilling a phosphorus oxychloride reaction product, the molar ratio of phosphorus pentachloride:phosphonic dichloride being from 0.1:1 to 10:1 is disclosed.

There is further disclosed a process for chlorinating compounds containing a carbon atom bonded to a hetero-atom selected from oxygen, nitrogen and sulfur which comprises contacting the carbon hetero-atom containing compound with a chloro phosphorus complex corresponding to the following general formula:

$$\overset{\oplus}{RPCl_3}\overset{\ominus}{PCl_6}$$

wherein R represents optionally substituted aryl or optionally substituted alkyl, so that the hetero-atom is replaced by at least one chlorine atom; the reaction may be extended to compounds containing the carbon to chlorine bond alpha to a carbon atom having at least one hydrogen atom, it may be further dehydrochlorinated by either heating to a temperature of from 50 to 300°C or by reaction with a selected base.

EP 0 283 270 A2

## Description

### PROCESSES USING A PHOSPHORUS COMPLEX

This invention relates to processes using a phosphorus complex; more particularly it relates to a process for chlorinating and dehydrochlorinating certain organic compounds and, specifically, to a process for preparing and using a phosphorus complex, e.g., phenyltrichlorophosphonium hexachlorophosphate as a reactant.

The phosphorus complex useful in the process of this invention is exemplified by the compound phenyltrichlorophosphonium hexachlorophosphate. This compound is known in the art and has heretofore been used in the preparation of certain phosphorus-containing compounds.

The article entitled "Reaction of Phosphorus Pentachloride With Unsaturated Hydrocarbons" by G.K. Fedorova et al., Zhur. Obshchei. Khim. 30 4044 (1960), reported the reaction of styrene with phosphorus pentachloride to form a colorless complex with the structure

$$C_6H_5CH=CHP^{\oplus}Cl_3PCL_6^{\ominus}$$

This reference further disclosed that styrylphosphonous dichloride is formed when the complex is reacted with $SO_2$. With styrene, the complex forms styrylphosphorus tetrachloride, and on reduction with red phosphorus, the complex is converted into styryldichlorophosphine. However, this reference does not disclose the unique processes of this invention.

In the article entitled "Tetrachloro-(m- and p-nitrophenyl) Phosphoranes" by I.N. Zhmurova et al., Org. Phos. Comp. Ref., 705, Vol. 3, Monatsh. 70, 1-19 (1937), the reaction disclosed in the previous reference was cited for preparing a phosphorus complex. This reference disclosed the treatment of

$$\text{m- and p-}NO_2C_6H_4P^{\oplus}Cl_3PCl_6^{\ominus}$$

with red phosphorus at a molar ratio of 3:2 to obtain tetrachloro-(nitrophenyl)phosphoranes. It was further stated therein that m- and p- nitrophenylphosphonic dichlorides required more severe reaction conditions than in the case of arylphosphonic dichlorides which do not contain a nitro group. In particular, it stated that phenyl and p-tolyl-phosphonic dichlorides react with phosphorus pentachloride in boiling benzene, but m-and p-nitrophenylphosphonic dichlorides react only when heated with phosphorus pentachloride at 150°C in absence of solvent. This reference further discloses that when nitrophenylphosphonic dichloride and phosphorus pentachloride were heated with a condenser set for distillation and a receiver cooled to -80°C for phosphoryl chloride, the yield realized was only about 65 to about 75 weight percent. It is noted that in the course of the reaction only a small amount of phosphorus oxychloride was distilled off and the reaction was carried out in the absence of a solvent. The improvements disclosed by the present invention are not disclosed by this reference.

U.S. Pat. 4,634,771, Shim et al., Jan. 6, 1987, disclosed the reaction of carboxylic acid groups on aromatic ring compounds with phenylphosphonous dichloride, chlorine, and phosphorus trichloride to convert the carboxylic acid groups to trichloromethyl groups. This reference distinguished the use of phosphorus pentachloride and suggested that phosphorus pentachloride is specific for the conversion of a carboxylic acid group adjacent, or alpha, to the hetero-atom of an N-heteroaromatic (N = nitrogen) compound. However, this reference did not disclose the use of the phosphorus complex exemplified by phenyltrichlorophosphonium hexachlorophosphate that is used in the process of this invention.

U.S. Pat. 4,419,514, McKendry et al., Dec. 6, 1983, disclosed a method for converting carboxylic acid groups to trichloromethyl groups which comprises contacting a compound containing a carboxylic acid group with phenylphosphonic dichloride and phosphorus pentachloride at a specified molar ratio for a time and at a temperature sufficient to carry out the conversion of a carboxylic acid group to a trichloromethyl group. However, this reference does not disclose the use of a phosphorus complex as the chlorinating agent. In fact, this reference teaches that it is helpful to allow the carboxylic acid compound and the phenylphosphonic dichloride compound to react for a period of time (until HCl evolution ceased) before adding the phosphorus pentachloride.

U.S. Pat. 4,167,525, Kataoka et al., Sept. 11, 1979, disclosed the preparation of aromatic acyl chlorides by reacting an aromatic carboxylic acid, phosphorus trichloride, and chlorine to produce phosphorus pentachloride in situ which is then treated with either water or certain phosphorus compounds to convert the phosphorus pentachloride into phosphorus oxychloride. The resulting reaction mixture is then distilled to obtain phosphorus oxychloride and, successively, the desired aromatic acyl chloride. The process of this

reference improves prior known processes by allowing the preparation of the aromatic acyl chloride without causing blocking of the distillation equipment by phosphorus pentachloride and without formation of troublesome by-products. However, this reference does not disclose the use of a phosphorus complex as disclosed in the process of this invention.

An object of this invention is to provide a process for simply chlorinating compounds containing a carbon atom bonded to a hetero-atom selected from the group consisting of oxygen, nitrogen and sulfur. A further object of this invention is to provide a process for reacting compounds containing a carbon atom alpha to a carbon atom containing at least one hydrogen atom and bonded to a hetero-atom selected from the group consisting of oxygen, nitrogen and sulfur.

An object of this invention is to provide a process for preparing a phosphorus complex, exemplified by phenyltrichlorophosphonium hexachlorophosphate, in substantially quantitative yields.

An embodiment of this invention is an improved process for preparing a phosphorus complex of the formula:

$$\overset{\oplus}{RPCl_3}\overset{\ominus}{PCl_6}$$

wherein R is selected from the group consisting of: aryl; substituted aryl, wherein the substituent can comprise at least one member selected from the group consisting of nitro, chloro, fluoro, alkyl, fluoro-alkyl, alkoxy and mixtures thereof; alkyl and substituted alkyl by the reaction of phosphorus pentachloride and a phosphonic dichloride of the formula:

$RPOCl_2$

wherein R is defined as above, wherein the improvement comprises: reacting the phosphorus pentachloride and the phosphonic dichloride in the presence of an inert solvent capable of co-distilling a phosphorus oxychloride reaction product and co-distilling said phosphorus oxychloride reaction product during the reaction of the phosphorus pentachloride and the phosphonic dichloride. In this process, a preferred solvent boils at a temperature ranging from about 120°C to about 150°C and the preferred phosphorus complex is phenyltrichlorophosphonium hexachlorophosphate.

A further embodiment of this invention is a process for chlorinating a compound containing a carbon atom bonded to a heter-atom selected from the group consisting of oxygen, nitrogen and sulfur, which comprises contacting the compound with a chloro phosphorus complex of the formula

$$\overset{\oplus}{RPCl_3}\overset{\ominus}{PCl_6}$$

wherein R is selected from the group consisting of aryl, substituted aryl, alkyl and substituted alkyl so that the hetero-atom is replaced by at least one chlorine atom.

This invention further discloses a process for reacting a compound containing a carbon atom alpha to a carbon atom containing at least one hydrogen atom and bonded to a hetero-atom selected from the group consisting of oxygen, nitrogen and sulfur which comprises

(a) contacting the compound with a chloro phosphorus complex of the formula

$$\overset{\oplus}{RPCl_3}\overset{\ominus}{PCl_6}$$

wherein R is selected from the group consisting of aryl, substituted aryl, alkyl and substituted alkyl so that the hetero-atom is replaced by at least one chlorine atom. The reaction can take place with or without the use of a non-reactive suitable solvent. Suitable solvents must of necessity be at the reaction conditions, high boiling and the reactants must be soluble therein. For example, chlorobenzenes and the like are suitable.

(b) The process may be extended to dehydrochlorinate the chlorine-containing compound formed in step (a). In this process, the chlorine-containing compound can be dehydrochlorinated by heating to a reaction temperature ranging from about 50° to about 300°C or, alternatively, by contacting the compound containing said bond with a base preferably selected from the group consisting of alkali metal hydroxides and alkoxides. The dehydrochlorination reaction may take place with or without a suitable solvent.

This invention discloses a process for preparing a phosphorus complex of the formula

$$R\overset{\oplus}{P}Cl_3.\overset{\ominus}{P}Cl_6 .$$

In this complex, R is selected from the group consisting of: $C_6$-$C_{12}$ aryl; $C_6$-$C_{12}$ substituted aryl; $C_1$-$C_6$ alkyl; and $C_1$-$C_6$ substituted alkyl. The substituents on the substituted aryl can comprise at least one member selected from the group consisting of nitro, chloro, fluoro, $C_1$-$C_6$ alkyl, $C_1$-$C_6$ fluoro-alkyl, $C_1$-$C_6$ alkoxy, and mixtures thereof. The substituents on the substituted alkyl can be selected from the group consisting of nitro, chloro, fluoro, and mixtures thereof. A preferred phosphorus complex is when R is phenyl with the resulting complex being phenyltrichlorophosphonium hexachlorophosphate.

It has heretofore been known to prepare the above disclosed phosphorus complex by the reaction of phosphorus pentachloride and a phosphonic dichloride of the formula: $RPOCl_2$, wherein R is defined as above.

This invention disclosed an improvement on this heretofore known complex formation process. In the complex formation process of this invention, the phosphorus pentachloride and the phosphonic dichloride are reacted in the presence of an inert solvent capable of co-distilling a phosphorus oxychloride reaction product. The following equation describes the reaction:

$$RPOCl_2 + 2PCl_5 \xrightarrow{\text{solvent}} R\overset{+}{P}Cl_3.\overset{-}{P}Cl_6 + POCl_3 .$$

The solvent useful in this reaction must be inert to the reactants and, further, must be capable of co-distilling the phosphorus oxychloride reaction product. Suitable solvents can include those solvents that boil at a temperature ranging from about 120° C to about 150° C. A preferred solvent is chlorobenzene, which boils at 132° C.

It is important in this complex formation process that the solvent be used to co-distill the phosphorus oxychloride reaction product during the reaction of the phosphorus pentachloride and the phosphonic dichloride. This is distinct from the usual practice of separating the solvent from the reaction products after the reaction is completed or, in the alternative, carrying out the reaction under reflux conditions.

The primary reaction between the phosphorus pentachloride and the phosphonic dichloride can be carried out at a temperature ranging from about 75° C to about 175° C, with a temperature ranging from about 120° C to about 135° C being preferred.

The molar ratio of the reactants, i.e., phosphorus pentachloride to phosphonic dichloride can range from about 0.1:1 to about 10:1 However, the stoichiometric ratio of 2:1 is preferred for economic reasons. The reaction can be carried out at atmospheric pressure.

As heretofore practiced, the reaction between the phosphorus pentachloride and the phosphonic dichloride was carried out with or without a solvent, and especially without the careful selection of a particular solvent. Additionally, the concurrent distillation of the resulting phosphorus oxychloride reaction product was neither disclosed nor suggested. The improvement resulting from following the additional steps of the process of this invention includes the preparation of the resulting phosphorus complex in substantially quantitative yield. This is compared to the prior known process wherein the yields usually realized range from about 60 to about 75 weight percent. In this process, the yields should range from above 80 weight percent to a substantially quantitative yield.

In the dehydrochlorination process of this invention, the hetero-atom in a carbon to hetero-atom bond is replaced with a chlorine atom to form a carbon to chlorine bond. This compound containing the carbon to chlorine bond can further be dehydrochlorinated when said compound contains a second carbon atom containing at least one hydrogen atom, alpha to the first carbon atom.

The compound containing the carbon atom bonded to the hetero-atom is exemplified by the listing of compounds in the following Table. The Table further lists the resulting chlorinated compound and, where appropriate, the resulting dehydrochlorinated compound.

TABLE 1

| No. | C-X Compounds | C-Cl Compounds | Dehydrochlorinated Compounds |
|---|---|---|---|
| 1 | $R^1$-OH | $R^1$-Cl | |
| 2 | 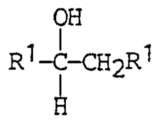 | 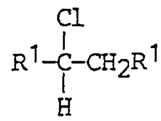 | 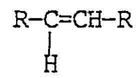 |
| 3 | $R^1$-COOH | $R^1CCl_3$ | |
| 4 | $R^1$-O-$R^1$ | $R^1$-Cl | |
| 5 | 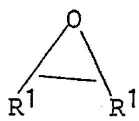 | 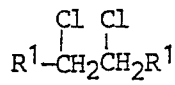 | $R^1CH{=}CHR^1$ & $R^1C{\equiv}CR^1$ |
| 6 | $R^1$-$NO_2$ | $R^1$-Cl | |
| 7 | 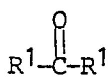 | $R^1CCl_2R^1$ | |
| 8 | 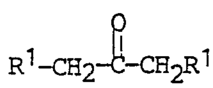 | 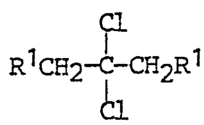 | 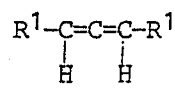 |
| 9 | 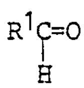 | $R^1CCl_2H$ | |
| 10 | 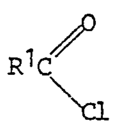 | $R^1CCl_3$ | |
| 11 | 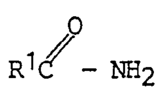 | 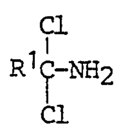 | $R^1CN$ |
| 12 | 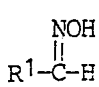 | | $R^1CN$ |
| 13 | 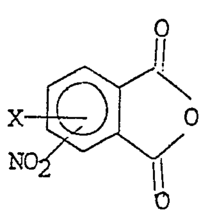 | 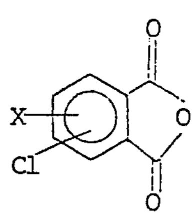 | |

$R^1$ in the above table is selected independently each time it occurs from the group consisting of $C_1$-$C_{24}$

straight and branched chained alkyl, $C_6$-$C_{10}$ aryl, and $C_6$-$C_{10}$ aryl substituted with one or more alkyl groups. The compounds listed in the above table are merely exemplary and are not meant to be inclusive of all compounds. The compounds listed under the column designated "C-X Compounds", exemplify certain compounds containing a carbon atom bonded to a hetero-atom selected from the group consisting of oxygen, nitrogen and sulfur. The compounds listed under the column designated "C-Cl Compounds" represent certain of the chlorinated compounds that can result from the process of this invention. The compounds listed under the column designated "Dehydrochlorinated Compounds" represent some of the compounds that can result from the process of this invention.

In this invention, the compound containing the carbon atom bonded to the hetero-atom is chlorinated by contacting said compound with a chlorophosphorus complex. This chloro phosphorus complex is of the formula

$$R\overset{\oplus}{P}Cl_3\overset{\ominus}{P}Cl_6$$

wherein R is selected from the group consisting of $C_6$-$C_{10}$ aryl; $C_6$-$C_{12}$ substituted aryl, wherein the substituents can comprise at least one member selected from the group consisting of nitro, chloro, fluoro, $C_1$-$C_6$ alkyl, $C_1$-$C_6$ fluoroalkyl, $C_1$-$C_6$ alkoxy and mixtures thereof; $C_1$-$C_6$ alkyl; and $C_1$-$C_6$ substituted alkyl. The substituents on the substituted alkyl can be selected from the group consisting of nitro, chloro, fluoro and mixtures thereof. The substituents listed above for both the substituted aryl and the substituted alkyl are merely preferred substituents and can include any non-interfering substituents. A preferred phosphorus complex results when R is phenyl with the resulting complex being phenyltrichlorophosphonium hexachlorophosphate. These chlorophosphorus complexes can be prepared by any of the known methods, but it is especially preferred to use the process described in the parent application.

The reaction of the chlorophosphorus complex with the compound containing the carbon to hetero-atom bond can take place in the presence of a solvent. A preferred solvent is phenylphosphonic dichloride. Additionally, the reaction can take place at various temperatures, depending upon the particular reactants. Increased temperatures will generally increase the rate of reaction and reduce the actual reaction time.

The compounds capable of being chlorinated by this chlorophosohorus complex are varied. When the hetero-atom is oxygen, these compounds can include alcohols, carboxylic acids, ketones, aldehydes, acetals, ketals, ethers, epoxides, acid chlorides, esters and mixtures thereof. When the hetero-atom is nitrogen, the compounds can include nitro-aromatics, amides, oximes and mixtures thereof. When the hetero-atom is sulfur, the compounds can include thiols, sulfoxides, sulfones, sulfonates and sulfides.

This invention further discloses a process for reacting a compound containing a carbon atom alpha to a carbon atom containing at least one hydrogen atom and bonded to a hetero-atom selected from the group consisting of oxygen, nitrogen and sulfur. In this process, this compound is first contacted with the chloro-phosphorus complex described above so that the hetero-atom is replaced by at least one chlorine atom, and then the carbon chlorine bond formed is dehydrochlorinated. When the hetero-atom is oxygen, the compound is dehydrated. When the hetero-atom is nitrogen, ammonia is eliminated, and when the hetero-atom is sulfur, hydrogen sulfide is eliminated.

The carbon chlorine bond formed can be dehydrochlorinated by any known method. In particular, this bond can be dehydrochlorinated by heating the reaction mixture to a reaction temperature ranging from about 50°C to about 300°C. Additionally, the dehydrochlorination can be accomplished by contacting the compound containing the carbon chlorine bond with a base. The base is preferably selected from the group consisting of alkali metal hydroxides and alkali metal alkoxides.

The following experiments describe various embodiments of this invention.

## EXPERIMENTS

A. Preparation of Phenyltrichlorophosphonium hexachlorophosphide (complex)

A solution of chlorobenzene (150 ml) and phosphorus trichloride (73 g, 0.532 mole) was placed in a 500 ml four-necked flask under a nitrogen atmosphere. The flask was fitted with an overhead stirrer, reflux condenser, thermometer, and gas inlet tube. Chlorine gas (38 g, 0.535 mole) was blown onto the surface of the stirred solution at a rate of 1 g/min. The reaction temperature increased to 70°C as the chlorine reacted with the phosphorus trichloride to prepare phosphorus pentachloride. The gas inlet tube was replaced with an addition funnel after the chlorine addition was completed.

Phenylphosphonic dichloride (50 g, 0.256 mole) was added through the addition funnel over a 10-minute period to the stirred slurry of phosphorus pentachloride at 70°C. The reaction mixture was heated to 120°C for two hours. The reflux condenser was replaced with a distilling head and the reaction heated to boiling (~132°C) for about two hours. During this two-hour time period, the reaction by-product, phosphorus oxychloride, and about half of the solvent were co-distilled. The reaction mixture was then cooled to room temperature. The reaction flask was stoppered and placed in a nitrogen-filled glove box. The reaction mixture

was filtered through a sintered glass funnel in the glove box. The solid product was placed in a vacuum dessicator and the last traces of solvent removed under vacuum at room temperature. The dry weight of the resulting phenyltrichlorophosphonium hexachlorophosphide was 106 g, representing a yield of 90 weight percent.

1. Trichlorophenylphosphonium hexachlorophosphide

$$(C_6H_5\overset{\oplus}{P}Cl_3\overset{\ominus}{P}Cl_6)$$

was prepared according to either the procedure disclosed by Fedorova et al. in Zhur. Obshchei. Khim 30, 4044 (1960) or the procedure disclosed in U.S. Patent Application Serial No. 026311, filed March 16, 1986, entitled "Preparation of a Phosphorus Complex.", also described hereinabove in Experiment A.

2. Representative Reaction of Aromatic Nitro Compounds with Trichlorophenylphosphonium Hexachlorophosphide

B. Nitrobenzene

To a mixture of trichlorophenylphosphonium hexachlorophosphide (18.5 g, 40.6 millimoles) in the solvent phenylphosphonic dichloride (100 ml) was added nitrobenzene (5.0 g, 40.6 mM). The yellow reaction mixture was heated to about 160°C for 12 hours. After cooling to room temperature, the reaction mixture was poured onto ice and the resulting product extracted with ether three times. The combined organic extracts were washed with water and brine and dried over anhydrous magnesium sulfate. Removal of the ether by distillation gave chlorobenzene (4.2 g, 92 wt. % yield) as a yellow liquid.

C. 3-Nitrophthalic Anhydride

Phenyltrichlorophosphonium hexachlorophosphide (4 g, 8.8 mM) was added to phenylphosphonic dichloride (10 ml). To this solutoin, 3 nitrophthalic anhydride (1.7 g, 8.8 mM) was added. The reaction mixture was heated to 170°C for 12 hours, then cooled to room temperature and poured onto ice. After neutralizing with 50% aqueous sodium hydroxide, the reaction was extracted with ethyl acetate. The combined ethyl acetate extracts were washed with brine, dried over magnesium sulfate, and concentrated in vacuo to give 3-chlorophthalic anhydride (1.3 g, 81 wt. % yield) as a tan solid.

3. Representative Reaction of Alcohols with Trichlorophenylphosphonium Hexachlorophosphide

To a mixture of trichlorophenylphosphonium hexachlorophosphide (10.5 g, 22.9 mM) in the solvent phenylphosphonic dichloride (50 ml) was added 3-methyl-1-butanol (2.02 g, 22.9mM). The reaction mixture was stirred at room temperature for 12 hours and worked-up as described above to give 3-methyl-1-chlorobutane (2.2 g, 91 wg. % yield) as a clear liquid.

4. Representative Reaction of Epoxides with Trichlorophenylphosphonium Hexachlorophosphide

To a mixture of trichlorophenylphosphonium hexachlorophosphide (3.8 g, 8.3 mM) in the solvent phenylphosphonic dichloride (10 ml) was added 1,2-epoxyhexane (831 mg, 8.3 mM). The reaction mixture was stirred for 12 hours at room temperature and worked-up as described above to 1,2 dichlorohexane (678 mg, 53 wt. % yield) as a yellow liquid.

5. Representative Reaction of Carboxylic Acids with Trichlorophenylphosphonium Hexachlorophosphide

To a mixture of trichlorophenylphosphonium hexachlorophosphide (10.2 g, 22.3 mM) in the solvent phenylphosphonic dichloride (50 ml) was added benzoic acid (2.5 g, 22.3 mM). The reaction mixture was heated to 170°C for 48 hours and worked-up as described above to give trichloromethyl benzene (4.2 g, 98 wt. % yield) as a yellow liquid.

**Claims**

1. A process for the preparation of a phosphorus complex corresponding to the following general formula:

$$\overset{\oplus}{RPCl_3}\overset{\ominus}{PCl_6}$$

wherein R represents optionally substituted aryl, wherein the substitution may comprise at least one selected from nitro, chloro, fluoro, alkyl, fluoro-alkyl and alkoxy, or optionally substituted alkyl; characterised in that it comprises reacting phosphorus pentachloride and a phosphonic dichloride corresponding to the following general formula:

$RPOCl_2$

wherein R is as defined above; in the presence of an inert solvent boiling at a temperature of from 120 to 150°C, which is capable of co-distilling a phosphorus oxychloride reaction product, and co-distilling a phosphorus oxychloride reaction product, the molar ratio of phosphorus pentachloride:phosphonic dichloride being from 0.1:1 to 10:1.

2. A process as claimed in claim 1 wherein the solvent is chlorobenzene, the reaction is carried out at a temperature of from 120 to 135°C and the molar ratio of phosphorus pentachloride:phosphonic dichloride is 2:1.

3. A process for the chlorination of a compound containing a carbon atom bonded to a hetero-atom selected from oxygen, nitrogen and sulfur characterised in that it comprises contacting the compound with a chlorophosphorus complex corresponding to the following general formula:

$$\overset{\oplus}{RPCl_3}\overset{\ominus}{PCl_6}$$

wherein R represents optionally substituted aryl, or optionally substituted alkyl; optionally in the presence of a solvent, the hetero-atom being replaced by at least one chlorine atom.

4. A process as claimed in claim 3 wherein the hetero-atom is oxygen and the compound is selected from alcohols, carboxylic acids, ketones, aldehydes, acetals, ketals, ethers, epoxides, acid chlorides, esters and mixtures thereof; or wherein the hetero-atom is nitrogen and the compound is selected from nitro-aromatics, amides, oximes and mixtures thereof; or wherein the hetero-atom is sulfur and the compound is selected from thiols, sulfoxides, sulfones, sulfonates and sulfides.

5. A process as claimed in claim 4 wherein a nitroaromatic is used which is a nitrophthalic anhydride corresponding to the following general formula:

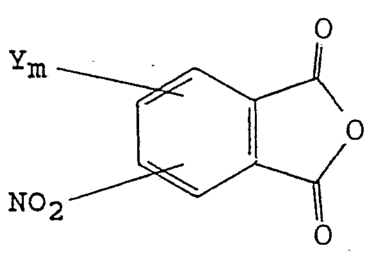

wherein m represents 0, 1, 2 or 3; and Y independently represents alkyl, alkoxy, haloalkyl or halogen.

6. A process for reacting a compound containing a carbon atom alpha to a carbon atom having at least one hydrogen atom and bonded to a hetero-atom selected from oxygen, nitrogen and sulfur characterised in that it comprises:

(a) contacting the compound with a chlorophosphorus complex corresponding to the following general formula:

$$\overset{\oplus}{RPCl_3}\overset{\ominus}{PCl_6}$$

wherein R represents optionally substituted aryl or optionally substituted alkyl; the hetero-atom being replaced by at least one chlorine atom; and

(b) dehydrochlorinating the resulting carbon-chlorine bond by heating to a temperature of from 50

to 300° C, or by contacting with a base.

7. A process as claimed in claim 6 wherein the compound is selected from alcohols, carboxylic acids, ketones, aldehydes, acetals, ketals, ethers, epoxides, acid chlorides, esters and mixtures thereof; nitro-aromatics, amides, oximes and mixtures thereof or thiols.

8. A process as claimed in claim 6 or claim 7 wherein a solvent is present.